Europäisches Patentamt

European Patent Office

Office européen des brevets

(19)

(11) Veröffentlichungsnummer: **0 001 252**
**B1**

(12)

# EUROPÄISCHE PATENTSCHRIFT

(21) Anmeldenummer: **78100866.9**

(22) Anmeldetag: **12.09.78**

(51) Int. Cl.³: **C 07 C  63/10,** C 07 B  9/00

(54) Verfahren zur Herstellung von m-Halogenbenzoylhalogeniden

(30) Priorität: **24.09.77 DE 2743144**

(43) Veröffentlichungstag der Anmeldung:
**04.04.79 Patentblatt 79/07**

(45) Bekanntmachung des Hinweises auf die Patenterteilung:
**06.08.80 Patentblatt 80/16**

(84) Benannte Vertragsstaaten:
**CH DE FR GB NL**

(56) Entgegenhaltungen:
**GB - A - 714 206**

(73) Patentinhaber: **Hoechst Aktiengesellschaft**
**Postfach 80 03 20**
**D - 6230 Frankfurt**
**Main 80 (DE)**

(72) Erfinder: **Landauer, Franz, Dr.**
**Liederbacher Strasse 7**
**D - 6230 Frankfurt**
**Main 80 (DE)**

Courier Press, Leamington Spa, England.

# 0 001 252

## Verfahren zur Herstellung von m-Halogenbenzoylhalogeniden

m-Halogenbenzoylhalogenide sind wertvolle Ausgangs- und Zwischenprodukte für die Herstellung von Farbstoffen, Pharmazeutika und Pflanzenschutzmitteln. Insbesondere das m-Chlorbenzoylchlorid ist ein wichtiges Ausgangsmaterial für die Herstellung von Herbiziden auf der Basis der Halophenoxybenzoesäure, die in der US—PS 3 652 645 beschrieben ist.

Zur Herstellung von m-Halogenbenzoylhalogeniden ist eine Reihe verschiedener Verfahren bekannt. Ein vorteilhaftes Verfahren zur Herstellung von m-Chlorbenzoylchlorid besteht beispielsweise in der Chlorierung von Benzoylchlorid in Abwesenheit eines Lösungsmittels und in Gegenwart von wasserfreiem $FeCl_3$ als Katalysator und von Jod als Cokatalysator bei einer Temperatur zwischen 0 und 50°C (DE—AS 2 538 158). Die hierbei gemäß den Beispielen erzielten Ausbeuten an m-Chlorbenzoylchlorid zwischen etwa 60 und 66%, bezogen auf umgesetztes Benzoylchlorid, erscheinen jedoch — insbesondere für großtechnische Anforderungen — nicht ganz befriedigend und daher noch verbesserungsbedürftig. Außerdem verursacht das als Cokatalysator verwendete Jod oft unerwünschte Verfärbungen des Verfahrensprodukts.

Es war daher wünschenswert und bestand die Aufgabe, das erwähnte bekannte Verfahren so abzuändern bzw. zu verbessern, daß höhere Ausbeuten und keine verfärbten Produkte mehr erhalten werden. Diese Aufgabe konnte erfindungsgemäß in einfacher und befriedigender Weise dadurch gelöst werden, daß man anstelle des Jods als Cokatalysator bei dem Verfahren der DE—AS 2 538 158 Schwefel und/oder mindestens eine anorganische und/oder organische S-Verbindung verwendet. Das Verfahren ist nicht nur anwendbar auf die Herstellung von m-Chlorbenzoylchlorid, sondern auch auf die Herstellung von m-Brombenzoylchlorid sowie von m-Chlorbenzoylbromid und m-Brombenzoylchlorid. Erfindungsgegenstand ist somit ein Verfahren zur Herstellung von m-Halogenbenzoylhalogeniden, wobei Benzoylchlorid oder -bromid in Abwesenheit eines Lösungsmittels und in Gegenwart von $FeCl_3$ oder $FeBr_3$ als Katalysator und eines Cokatalysators bei einer Temperatur zwischen etwa 0 und 90°C, vorzugsweise zwischen etwa 10 und 75°C, chloriert oder bromiert wird; das Verfahren ist dadurch gekennzeichnet, daß man als Cokatalysator Schwefel und/oder mindestens eine anorganische und/oder organische S-Verbindung verwendet.

Wie schon vorher erwähnt, ist es möglich, nach dem Verfahren sowohl m-Halogenbenzoylhalogenide mit gleichem Halogen im Molekül (m-Chlorbenzoylchlorid und m-Brombenzoylbromid) als auch gemischte m-Halogenbenzoylhalogenide (m-Chlorbenzoylbromid und m-Brombenzoylchlorid) herzustellen; bevorzugt ist die Herstellung von m-Chlorbenzoylchlorid. Es ist vorteilhaft, im Falle der Chlorierung als Katalysator $FeCl_3$ und im Falle der Bromierung als Katalysator $FeBr_3$ zu verwenden, doch ist im Prinzip auch die Verwendung von $FeBr_3$ bei der Chlorierung und von $FeCl_3$ bei der Bromierung möglich. Im Falle der Herstellung von gemischten Halogenverbindungen können $FeCl_3$ und $FeBr_3$ in gleicher Weise eingesetzt werden. Die Katalysatoren sollen möglichst wasserfrei sein. Als Cokatalysator wird erfindungsgemäß Schwefel und/oder mindestens eine anorganische und/oder organische S-Verbindung verwendet. Als organische S-Verbindungen kommen in erster Linie Schwefelhalogenide wie $SCl_2$ und $S_2Cl_2$ sowie die entsprechenden Bromide in Frage, wobei $S_2Cl_2$ und $S_2Br_2$ bevorzugt sind. Auch hier ist es — in gleicher Weise wie beim Katalysator — zweckmäßig, bei der Chlorierung ein Schwefelchlorid und bei der Bromierung ein Schwefelbromid zu verwenden.

Als organische Schwefelverbindungen kommen hauptsächlich organ. Verbindungen des 2-wertigen Schwefels mit noch 1 freien H-Atom am Schwefel wie z.B. Thioglykolsäure, Thioessigsäure, Thiophenol etc. in Frage, doch ist auch die Verwendung 2-wertiger organ. S-Verbindungen ohne freies H-Atom am S, wie z.B. von Thiophen, Thioharnstoff etc. möglich. Es kann sowohl Schwefel oder eine der genannten anorganischen oder organischen Schwefelverbindungen allein oder auch eine Mischung der genannten Cokatalysator-substanzen eingesetzt werden. Bevorzugt ist die Chlorierung von Benzoylchlorid in Gegenwart von $FeCl_3$ als Katalysator sowie von S und/oder $S_2Cl_2$ als Cokatalysator.

Die Katalysatormenge beträgt zweckmäßig etwa 0,05 bis 2 Gew.-%, vorzugsweise etwa 0,1 bis 1 Gew.-%, bezogen auf das Ausgangsbenzoylchlorid oder -bromid.

Der Cokatalysator soll zweckmäßig in einer Menge entsprechend einem S-Gehalt von etwa 0,01 bis 2 Gew.-%, vorzugsweise etwa 0,05 bis 0,5 Gew.-%, bezogen auf das Ausgangsbenzoylchlorid oder -bromid, zugegen sein. Das zur Halogenierung verwendete Chlor oder Brom kann sowohl flüssig als auch gasförmig eingesetzt werden. Die Reaktion läßt sich sowohl drucklos als auch bei Über oder vermindertem Druck in bekannten Apparaturen sowohl diskontinuierlich als auch kontinuierlich durchführen. Selbstverständlich ist in bekannter Weise darauf zu achten, daß keine — oder zumindest keine wesentliche — Überchlorierung oder -bromierung stattfindet, da sonst die Ausbeute an dem gewünschten m-Halogenprodukt und damit die Wirtschaftlichkeit des Verfahrens beeinträchtigt wird.

Die nach der Erfindung erzielten Ausbeuten an m-Halogenbenzoylhalogeniden liegen bei etwa 75 bis 80% d.Th. und höher, bezogen auf umgesetztes Ausgangsbenzoylchlorid bzw. -bromid, und sind damit etwa 10% und mehr höher als die in der DE—AS 2 538 158 angegebenen Ausbeuten. Bei der Destillation nach der Halogenierung werden farblose Destillate erhalten.

Es war zwar bekannt, bei der Halogenierung aromatischer Verbindungen $Fe^{III}$-Katalysatoren und S-haltige Cokatalysatoren zu verwenden (s. z.B. AU—PS 223 024, AU—PS 230 337 und DE—PS 1 543 030), doch dienten diese Katalysator — Cokatalysatorkombinationen ausschließlich der Erhöhung

# 0 001 252

des Verhältnisses von p- zu o-Isomeren bei der Herstellung von Dichlorbenzol (AU—PS 223 024, AU—PS 230 337) sowie von Monochlortoluol (DE—PS 1 543 020). Ausgehend von diesem Befund war es überraschend und in keiner Weise naheliegend, die gleiche Katalysator — Cokatalysatorkombination anzuwenden, wenn man vor dem Problem stand, durch Halogenierung von Benzoylhalogeniden höhere Ausbeuten an dem m-Halogenprodukt zu erhalten.

Die folgenden Beispiele sollen der weiteren Erläuterung der Erfindung dienen.

## BEISPIEL 1

In einem Vierhalskolben mit Rückflußkühler, Thermometer und Gaseinleitungsrohr werden 421 g Benzoylchlorid (3 Mol) eingefüllt. Nach Zugabe von 1,8 g Eisen-III-chlorid (= 0,43% vom Benzoylchlorid) und 0,9 g Schwefelpuder (= 0,21% vom Benzoylchlorid) werden bei 30—35°C in 3 Stunden 160 g Chlor (2,25 Mol) eingeleitet. Nach Ausblasen des Chlorwasserstoffs mit Stickstoff wird das Chlorierungsgemisch vom Katalysator abdestilliert, wobei der Siedebereich im Vakuum 930 Pa (7 mmHg) zwischen 60 und 93°C liegt. Es wurden 490 g Destillat erhalten. Nach der gaschromatographischen Analyse beträgt die Zusammensetzung:

|  | | Gew.-% | g | Mol |
|---|---|---|---|---|
| Benzoylchlorid | | 35,5 | 174,2 | 1,24 |
| m-Cl | " | 49,3 | 241,5 | 1,38 |
| p-Cl | " | 1,0 | 4,9 | 0,03 |
| q-Cl | " | 6,2 | 30,5 | 0,17 |
| Di-Cl | " | 7,7 | 37,8 | 0,18 |

Dies bedeutet, daß von den verbrauchten 1,76 Molen Benzoylchlorid 1,38 Mole der gewünschten m-Chlor-Verbindung erhalten wurden. Dies sind 78,4% d.Th..

Die gleiche Ausbeute wird erhalten, wenn die Chlorierung bei 20—25°C durchgeführt wird.

## BEISPIEL 2

In der gleichen Apparatur wie in Beispiel 1 angegeben werden 400 g Benzoylchlorid (2,85 Mol) nach Zugabe von 1,8 g Eisen-III-chlorid und 1,1 g Thioglykolsäure bei 35°C chloriert. Dabei werden im Verlauf von 2 Stunden 110 g Chlor (= 1,55 Mol) eingeleitet. Nach Ausblasen des Chlorwasserstoffs mit Stickstoff wird das Chlorierungsgemisch von den Katalysatoren durch Destillation im Vakuum gereinigt. Der Siedebereich liegt zwischen 92° und 125°C (bei 3600 Pa (27 mm Hg)). Es wurden 431 g Destillat erhalten.

Die gaschromatographische Analyse ergab folgende Zusammensetzung:

|  | | Gew.-% | g | Mol |
|---|---|---|---|---|
| Benzoylchlorid | | 50 | 215,6 | 1,53 |
| m-Cl- | " | 39 | 168 | 0,96 |
| p-Cl- | " | 0,9 | 3,4 | 0,02 |
| o-Cl- | " | 6,1 | 26,3 | 0,15 |
| Di-Cl- | " | 5,9 | 25,4 | 0,12 |

Dies bedeutet, daß von den verbrauchten 1,32 Molen Benzoylchlorid 168 g m-Chlorbenzoylchlorid (= 0.96 Mol), das sind 73% d.Th., erhalten wurden.

## BEISPIEL 3

In der gleichen Apparatur wie in Beispiel 1 angegeben werden zu 393 g Benzoylchlorid (= 2,8 Mol), 1,7 g FeCl$_3$ (0,43 Gew.-%) und 1,68 g Dischwefeldichlorid (= 0,43 Gew.-% bzw. 0,2 Gew.-% an Schwefel) im Verlauf von 2 Stunden 130 g Chlor (= 1,83 Mol) bei 30—40°C eingeleitet. Nach Ausblasen des Chlorwasserstoffs wird das Chlorierungsgemisch abdestilliert, wobei das Produkt zwischen 96° und 130°C (bei 3600 Pa (27 mm Hg)) übergeht. Es wurden 443 g Destillat erhalten.

Die gaschromatographische Analyse ergab folgende Zusammensetzung:

|  | Gew.-% | g | Mol |
|---|---|---|---|
| Benzoylchlorid | 42 | 186 | 1,31 |
| m-Cl- '' | 45 | 199,4 | 1,14 |
| p-Cl- '' | 1,0 | 4,4 | 0,03 |
| o-Cl- '' | 6,2 | 27,4 | 0,16 |
| Di-Cl- '' | 5 | 22,2 | 0,10 |

Die Ausbeute an m-Chlorbenzoylchlorid beträgt somit 76,5% d.Th., bezogen auf umgesetztes Bezoylchlorid.

## BEISPIEL 4

In einem Vierhalskolben mit Rückflußkühler, Thermometer und Tropftrichter werden 281 g Benzoylchlorid (= 2 Mol), 2,5 g Eisen-III-chlorid (= 0,9%) und 1,5 g Schwefelpuder (= 0,53%) vorgelegt. Bei 70—75°C werden dann im Verlauf von 4,5 Stunden 100 g Brom (= 0,63 Mol) zugetropft. Nach Ausblasen Bromwasserstoffs wird durch Vakuumdestillation von den Katalysatoren abgetrennt. Zwischen 95° und 145°C (bei 3600 Pa (27 mm Hg)) destillieren 325 g über. Durch gaschromatographische Analyse wurde gefunden:

|  | Gew.-% | g | Mol |
|---|---|---|---|
| Benzoylchlorid | 69 | 224,3 | 1,60 |
| m-Brom- '' | 30 | 97,5 | 0,44 |
| p-Brom- '' | 0,4 | 1,3 | 0,006 |
| o-Brom- '' | 0,4 | 1,3 | 0,006 |

Die Ausbeute an m-Brom-benzoylchlorid ist somit — be = zogen auf umgesetztes Benzoylchlorid — praktisch quantitativ.

## Patentansprüche

1. Verfahren zur Herstellung von m-Halogenbenzoylhalogeniden, wobei Benzoylchlorid oder -bromid in Abwesenheit eines Lösungsmittels und in Gegenwart von FeCl$_3$ oder FeBr$_3$ als Katalysator sowie eines Cokatalysators bei einer Temperatur zwischen etwa 0 und 90°C, vorzugsweise zwischen etwa 10 und 75°C, chloriert oder bromiert wird, dadurch gekennzeichnet, daß man als Cokatalysator Schwefel und/oder mindestens eine anorganische und/oder organische S-Verbindung verwendet.

2. Verfahren nach Anspruch 1, dadurch gekennzeichnet, daß man Benzoylchlorid in Gegenwart von FeCl$_3$ als Katalysator und von S und/oder S$_2$Cl$_2$ als Cokatalysator chloriert.

3. Verfahren nach Anspruch 1 bis 2, dadurch gekennzeichnet, daß man den Katalysator in einer Menge von etwa 0,05 bis 2 Gew.-%, vorzugsweise von etwa 0,1—1 Gew.-%, benzogen auf das Ausgangs-Benzoylchlorid oder -bromid, verwendet.

4. Verfahren nach Anspruch 1 bis 3, dadurch gekennzeichnet, daß man den Cokatalysator in einer Menge entsprechend einem S-Gehalt von etwa 0,01—2 Gew.-%, vorzugsweise etwa 0,05—0,5 Gew.-%, bezogen auf das Ausgangsbenzoylchlorid oder -bromid, verwendet.

## Claims

1. Process for the preparation of m-halogenobenzoyl halides by chlorinating or brominating benzoyl chloride or benzoyl bromide in the absence of a solvent and in the presence of FeCl$_3$ or FeBr$_3$ as the catalyst and of a cocatalyst, at a temperature of from about 0 to 90°C, preferably of from about 10 to 75°C, which comprises using as cocatalyst sulfur and/or at least one inorganic and/or organic sulfur compound.

2. Process as claimed in claim 1, which comprises chlorinating benzoyl chloride in the presence of FeCl$_3$ as the catalyst and of sulfur and/or S$_2$Cl$_2$ as the cocatalyst.

3. Process as claimed in claims 1 or 2, which comprises using the catalyst in an amount of from about 0.05 to 2 weight percent, preferably of from about 0.1 to 1 weight percent, calculated on benzoyl chloride or benzoyl bromide used as the starting compound.

4. Process as claimed in claims 1 to 3, which comprises using the cocatalyst in an amount corresponding to a sulfur content of from about 0.1 to 2 weight percent, preferably of from about 0.05 to 0.5 weight percent, calculated on benzoyl chloride or benzoyl bromide used as the starting compound.

## 0 001 252

**Revendications**

1. Procédé d'obtention d'halogénures de m-halogéno-benzoyle consistant à chlorer ou à bromer du chlorure de benzoyle ou du bromure de benzoyle en l'absence d'un solvant et en présence de $FeCl_3$ ou de $FeBr_3$ comme catalyseur et en présence d'un cocatalyseur, à une température entre environ 0 et 90°C, de préférence entre environ 10 et 75°C, procédé caractérisé en ce que l'on utilise comme cocatalyseur du soufre et/ou au moins un composé de soufre inorganique et/ou organique.

2. Procédé selon la revendication 1, caractérisé en ce que l'on chlore du chlorure de benzoyle en présence de $FeCl_3$ comme catalyseur et de S et/ou de $S_2Cl_2$ comme cocatalyseur.

3. Procédé selon l'une quelconque des revendications 1 et 2, caractérisé en ce que l'on utilise le catalyseur en une qualité d'environ 0,05 à 2% en poids, de préférence d'environ 0,1 à 1% en poids, par rapport au chlorure de benzoyle ou au bromure de benzoyle de départ.

4. Procédé selon l'une quelconque des revendications 1 à 3, caractérisé en ce que l'on utilise le cocatalyseur en une quantité correspondant à une teneur en soufre d'environ 0,01 à 2% en poids, de préférence d'environ 0,05 à 0,5% en poids, par rapport an chlorure de départ ou àu bromure de départ.